# EUROPÄISCHE PATENTSCHRIFT

(11) **EP 2 288 326 B1**
(45) Veröffentlichungstag und Bekanntmachung des Hinweises auf die Patenterteilung: **24.12.2014**
(21) Anmeldenummer: 09765529.4
(22) Anmeldetag: 30.05.2009
(51) Int. Cl.: A61J 1/03

(54) **PHARMAZEUTISCHE ZUBEREITUNG IN WASSERDAMPF DURCHLÄSSIGEN BEHÄLTERN MIT VERBESSERTER STABILITÄT**
PHARMACEUTICAL PREPARATION IN WATER VAPOUR ENCLOSURES WITH IMPROVED STABILITY
PREPARATION PHARMACEUTIQUE POSSEDANT UNE STABILITE AMELIOREE

(30) Priorität: 16.06.2008 DE 202008008079 U
(43) Veröffentlichungstag der Anmeldung: 02.03.2011
(73) Patentinhaber: G. Pohl-Boskamp GmbH & Co. KG, 25551 Hohenlockstedt (DE)
(72) Erfinder: GROTELÜSCHEN, Rolf, 24613 Aukrug (DE); UECK, Henning, 25524 Bekmünde (DE); ZIMMECK, Thomas, 25551 Hohenlockstedt (DE)
(74) Vertreter: Hamm, Volker
(86) Internationale Anmeldenummer: PCT/EP2009/003894
(87) Internationale Veröffentlichungsnummer: WO 2009/152949

(56) Entgegenhaltungen:
- EP-A1- 1 616 549
- WO-A1-97/38687
- US-A- 5 952 361
- US-A1- 2008 003 195

## Beschreibung

Die vorliegende Erfindung betrifft flüssige oder halbfeste, nicht-wässrige pharmazeutische Zubereitungen, die Alkohole und Triglyceride als Trägerstoffe enthalten, in Wasserdampf durchlässigen Behältern. Insbesondere betrifft die Erfindung pharmazeutische Zubereitungen enthaltend Nitroglycerin als Wirkstoff, die sich in einer Kunststoffflasche befinden.

Hilfsstoffe für pharmazeutische Anwendungen müssen hohen Anforderungen genügen. Neben der technologischen Sinnhaftigkeit und der Eignung für den Zweck ist die Unbedenklichkeit ein wichtiges Kriterium bei der Auswahl eines solchen Stoffes. Für nicht-wässrige Zubereitungen sind Alkohole und Triglyceride gebräuchliche Hilfsstoffe. Häufig verwendete Alkohole sind z.B. einwertige Alkohole wie Ethanol, 1- und 2-Propanol und die Butanole, aber auch zwei- und mehrwertige Alkohole wie Propylenglykol oder Glycerol. Gebräuchliche Triglyceride sind beispielsweise natürliche Öle und Fette sowie halbsynthetische Produkte wie mittelkettige Triglyceride, die z.B. unter dem Handelsnamen Miglyol® 812 vertrieben werden. gegenüber Wechselwirkungen mit dem Füllgut relativ inert, es ist durchsichtig und schützt den Inhalt vor äußeren Einflüssen. Es weist jedoch auch Nachteile auf, insbesondere die mangelnde Bruchsicherheit und das im Vergleich zu Kunststoff deutlich höhere Gewicht. Diese Nachteile treten besonders hervor bei Arzneimitteln, die Patienten auf Reisen oder ständig mit sich führen. Insbesondere die mangelnde Bruchsicherheit ist problematisch, da es in einer Notfallsituation leicht passieren kann, dass dem Patienten die Arzneimittelpackung mit der Glasflasche beziehungsweise Glasampulle aus der Hand fällt.

Als Alternative sind prinzipiell auch Behälter aus Kunststoff denkbar. Sie sind in jedem Fall leichter als Glasflaschen. Allerdings sind weitere Kriterien wie Bruchsicherheit, Transparenz und Inertheit gegenüber den Inhaltsstoffen zu erfüllen, was die Auswahl möglicher Kunststoffmaterialien einschränkt.

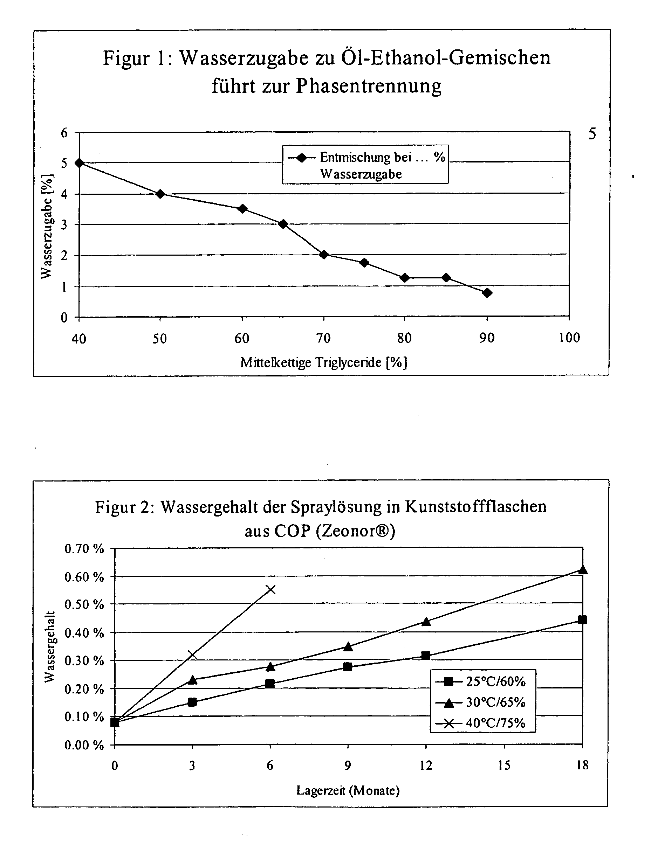

Im Rahmen der der vorliegenden Erfindung zugrunde liegenden Untersuchungen hat sich herausgestellt, dass ein weiteres Merkmal von großer Bedeutung ist: Öligalkoholische Zubereitungen nehmen aus der Umgebungsluft Feuchtigkeit in Form von Wasserdampf auf. Aufgrund der Affinität des Wassers zum Alkohol und den hydrophoben, also Wasser abstoßenden Eigenschaften des Öls kann es oberhalb eines bestimmten Wassergehalts zu einer Entmischung der Öl- und Alkoholkomponenten der Zubereitung kommen. Dabei bildet die Alkoholkomponente zusammen mit dem aufgenommenen Wasser eine erste Phase und das Öl (ggf. mit einer Restmenge des Alkohols) eine zweite Phase. Die maximale Menge an Wasser, die aufgenommen werden kann, bevor es zu einer Phasentrennung kommt, hängt von der Art der Alkohol- und Ölkomponente sowie von deren Mengenverhältnissen ab. Bei höheren Alkoholkonzentrationen kann mehr Wasser aufgenommen werden, bevor es zu einer Entmischung kommt. Beispielhafte Grenzkonzentrationen für Mischungen aus Ethanol und mittelkettigen Triglyceriden (Miglyol® 812), die zu einer Phasentrennung führen, sind in Figur 1 dargestellt. Dabei entsprechen die auf 100 % fehlenden Werte der X-Achse dem Ethanolgehalt der jeweils eingesetzten Lösung.

Für Arzneimittel wird aufgrund internationaler Richtlinien in der Regel gefordert, dass sie sechs Monate bei 40 °C und 75 % relativer Luftfeuchtigkeit und - je nach Klimazone - über die gesamte Haltbarkeitsdauer bei 30 °C und 65 % relativer Luftfeuchtigkeit beziehungsweise bei 25 °C und 60 % relativer Luftfeuchtigkeit stabil sind. Aus Gründen der Logistik und Vermarktung werden in der Regel mindestens 2 Jahre Haltbarkeitsdauer, bevorzugt 3 Jahre Haltbarkeitsdauer angestrebt. Diese Haltbarkeitsdauer wird jedoch in vielen Fällen ölig-alkoholischer Zubereitungen nicht erreicht. Es besteht daher ein Bedarf für Produkte in Kunststoffverpackungen, die diesen Anforderungen gerecht werden.

Aufgabe der vorliegenden Erfindung ist es somit, eine lagerstabile pharmazeutische Zubereitung umfassend ein Gemisch aus mindestens einer Alkoholkomponente und mindestens einer Triglyceridkomponente in einem Kunststoffbehälter anzugeben.

Gelöst wird diese Aufgabe durch die Merkmale der unabhängigen Ansprüche. Die abhängigen Ansprüche definieren vorteilhafte Ausführungsformen der Erfindung.

Es wurde überraschend gefunden, dass Kunststoffmaterialien mit einer Wasserdampfdurchlässigkeit von nicht mehr als 3,0 g/m²/24h (gemessen an einer Folie von 100 µm Stärke bei 40 °C und 90 % relativer Luftfeuchte) als Behälter für pharmazeutische Zubereitungen geeignet sind, die aufgrund ihres Gehalts an Triglyceriden und Alkoholen zu einer durch Wasser induzierten Entmischung neigen. Die erfindungsgemäßen Zubereitungen sind bei Lagerung über mindestens 6 Monate bei 40 °C und 75 % relativer Luftfeuchtigkeit stabil.

Bei der pharmazeutischen Zubereitung handelt es sich bevorzugt um eine flüssige oder halbfeste Zubereitung, besonders bevorzugt ist eine flüssige versprühbare Zubereitung. Die Zubereitung enthält eine Alkoholkomponente, eine Triglyceridkomponente sowie einen pharmazeutischen Wirkstoff, der sich vorzugsweise in der Zubereitung löst.

Die Alkoholkomponente wird bevorzugt aus der Gruppe bestehend aus Ethanol, 1-Propanol, 2-Propanol und Propylenglycol gewählt, wobei Ethanol besonders bevorzugt ist. Der bevorzugte Gehalt an Ethanol in den erfindungsgemäßen Zubereitungen liegt zwischen 10 und 80 Gew.-%, bezogen auf das Gesamtgewicht der Zubereitung.

Die Triglyceridkomponente wird bevorzugt aus der Gruppe der pflanzlichen und tierischen Fette und Öle gewählt. Besonders bevorzugt sind mittelkettige Triglyceride, die bevorzugt in einer Menge von 10 bis 80 Gew.-%, bezogen auf das Gesamtgewicht der Zubereitung, in den erfindungsgemäßen Zubereitungen enthalten sind.

Der in den erfindungsgemäßen Zubereitungen enthaltene Wirkstoff ist vorzugsweise Glyceroltrinitrat (GTN). Er ist bevorzugt in einer Menge von 0,2 bis 5 Gew.-%, bezogen auf das Gesamtgewicht der Zubereitung, in den erfindungsgemäßen Zubereitungen enthalten.

Bevorzugte Materialien für den Kunststoffbehälter der erfindungsgemäßen Zubereitungen sind Cycloolefinpolymere und Cycloolefincopolymere.

Die nachfolgenden Beispiele erläutern der Erfindung näher.

### Beispiel 1: Verpackung in Flaschen aus Polyethylenterephthalat (PET), Polycarbonat (PC) und Polypropylen (PP)

In diesem Beispiel wird eine Arzneistofflösung umfassend den Wirkstoff GTN, die nach dem bisherigen Stand der Technik in einer Glasflasche auf dem Markt ist, auf ihre Lagerstabilität in Kunststoffflaschen untersucht.

Für 1 kg der Arzneistofflösung werden 607 g mittelkettige Triglyceride, 7,2 g Pfefferminzöl, 200 g Ethanol abs., 20 g mittelkettige Partialglyceride und 166 g Glyceroltrinitrat (GTN) in mittelkettigen Triglyceriden (als 5 %-ige Lösung, enthaltend 8,3 g GTN) eingewogen und innig gemischt. Die Lösung wird in Portionen zu 14,9 g in 20 ml Kunststoffflaschen aus unterschiedlichen Materialien (PET (Polyethylenterephthalat), PC (Polycarbonat) und PP (Polypropylen)) abgefüllt, mit einer Spraypumpe verschlossen, einzeln gewogen und bei folgenden Lagerbedingungen gelagert: 25°C/60% relativer Feuchte; 30°C/60% relativer Feuchte und 40°C/75% relativer Feuchte. Die Flaschen werden nach ein und drei Monaten erneut gewogen.

Die folgende Tabelle zeigt die Gewichtsänderungen bei den einzelnen Flaschentypen:

| **Material** | **Prüfzeitpunkt** | **Lagerbedingung** | | |
|---|---|---|---|---|
| | | 25°C/60% | 30°C/60% | 40°C/75% |
| **PET** | 1 Mon. | 0,04 g | 0,06 g | 0,11 g |
| | 3 Mon. | 0,11 g | 0,13 g | 0,22 g |
| **PC** | 1 Mon. | 0,10 g | 0,12 g | 0,19 g |
| | 3 Mon. | 0,19 g | 0,22 g | 0,31g* |
| **PP** | 1 Mon. | <0,01 g | <0,01 g | *-0, 01 g¹⁾* |
| | 3 Mon. | 0,01 g | 0,01 g | *-0,01 g¹⁾* |

| | | | | |
|---|---|---|---|---|
| ^{*} Phasentrennung (Wassergehalt: 2,6 %) ¹⁾ Gewichtsverlust durch Verlust an Ethanol aus der Spraylösung | | | | |

Die Gewichtsänderung entspricht in erster Näherung der Wasseraufnahme aus der Umgebungsluft. Diese ist abhängig von der relativen Luftfeuchtigkeit. In Polycarbonat ist bereits nach drei Monaten die kritische Grenze überschritten und es kommt zu einer Phasentrennung. Flaschen aus PET nehmen etwa 30 - 50 % weniger Wasser aus der Umgebungsluft auf. In den Polypropylenflaschen wird bei der Lagerbedingung 40°C/75% Feuchte die Gewichtszunahme durch Wasser durch den Verlust an Ethanol ausgeglichen und durch Wiegen allein kann die Wasseraufnahme nicht ermittelt werden.

Dieses Beispiel zeigt, dass Polycarbonat als Verpackungsmaterial nicht geeignet ist. Für weitere Versuche wird die Wasseraufnahme nicht nur durch Differenzwägung ermittelt, sondern mittels Karl-Fischer-Titration ermittelt.

### Beispiel 2: Vergleich einer wirkstoffhaltigen und einer wirkstofffreien Zubereitung in PET-Flaschen

Für 1 kg der wirkstofffreien Zubereitung werden 773 g mittelkettige Triglyceride, 7,2 g Pfefferminzöl, 200 g Ethanol abs, und 20 g mittelkettige Partialglyceride eingewogen und innig gemischt. Die Lösung wird in Portionen zu 14,9 g in 20 ml Kunststoffflaschen aus PET abgefüllt, mit einer Spraypumpe verschlossen, und bei 40°C/75% relativer Feuchte eingelagert. Analog dazu werden Flaschen mit einer wirkstoffhaltigen Zubereitung nach Beispiel 1 abgefüllt und eingelagert.

Der Wassergehalt wird nach der Karl-Fischer-Methode des Europäischen Arzneibuchs unter Verwendung eines 787 KF Titrino (Firma Metrohm) bestimmt. Vor Beginn der Messung wird die Vorlage im Titriergefäß austitriert und die Drift bestimmt. Dann werden 1,0 bis 1,5 g Prüflösung mit einer Spritze in das Titriergefäß eingebracht. Das Gewicht wird mittels Rückwaage der Spritze ermittelt. Nach Austitrieren der Probe wird das Ergebnis in Prozent angegeben.

Die folgende Tabelle zeigt den jeweiligen Wassergehalt nach Lagerung bei 40 °C und 75 % rel. Luftfeuchte:

| Wassergehalt nach Lagerdauer von | 4 Wochen | 6 Wochen | 8 Wochen |
|---|---|---|---|
| Wirkstofffreie Zubereitung | 0,80% | | 1,24 % |
| Wirkstoffhaltige Zubereitung | | 1,27 % | 1,41 % |

Nach acht Wochen Lagerung bei erhöhter Luftfeuchtigkeit kommt es zu einer Phasentrennung. Daher ist die Flasche aus PET nicht geeignet.

### Beispiel 3: Vergleich weiterer Kunststoffmaterialien im Stresstest bei 40 °C und 75 % rel. Luftfeuchte

Die Lösung gemäß Beispiel 1 wird in Flaschen der folgenden Dimensionen und Materialien abgefüllt, verschlossen und bei 40°C/75% relativer Feuchte eingelagert.

| Material | Nennvolumen | Deckel | Füllmenge |
|---|---|---|---|
| Polyamid | 10 ml | Schraubdeckel | 6 g |
| Cycloolefincopolymer (Topas®) | 10 ml | Schraubdeckel | 6 g |
| Polypropylen | 15 ml | Spraypumpe | 12 g |

Zu unterschiedlichen Zeitpunkten wird der Wassergehalt nach der bei Beispiel 2 beschriebenen Methode bestimmt. Die erhaltenen Ergebnisse sind in der folgenden Tabelle zusammengefasst.

| Wassergehalt nach Lagerdauer von | 1 Monat | 3 Monate | 6 Monate |
|---|---|---|---|
| Polyamid | 0,90 % | * | |
| Cycloolefincopolymer (Topas®) | 0,26 % | 0,51 % | 0,84 % |
| Polypropylen | 0,31 % | 0,62 % | 0,94 % |

| | | | |
|---|---|---|---|
| * Der Versuch mit den Polyamidflaschen wurde nach einem Monat abgebrochen, da sich bereits zu diesem Zeitpunkt abzeichnete, dass der Wassergehalt innerhalb von 6 Monaten über 1 % steigen würde. | | | |

Dieser Versuch zeigt, dass auch Flaschen aus Polyamid die Anforderungen nicht erfüllen. Besonders geeignet sind Flaschen aus Cycloolefincopolymer (Topas®), da sie trotz des ungünstigeren Oberflächen-Volumen-Verhältnisses weniger Wasser aufnehmen als die Flaschen aus Polypropylen.

### Beispiel 4: Lagerung in Flaschen aus Cycloolefinpolymer bei unterschiedlichen Temperaturen

Die Lösung gemäß Beispiel 1 wird in Portionen zu je 12 g in 20 ml Flaschen aus Cycloolefinpolymer (Zeonor®), mit einer Spraypumpe verschlossen und bei 25°C/60% relativer Feuchte, bei 30°C/60% relativer Feuchte und bei 40°C/75% relativer Feuchte eingelagert. In dreimonatigen Abständen wird der Wassergehalt nach der bei Beispiel 2 beschriebenen Methode bestimmt. Die Ergebnisse sind in Figur 2 dargestellt.

Da die Wasseraufnahme annähernd linear verläuft, kann davon ausgegangen werden, dass der Wassergehalt der Zubereitung bei 30 °C/65 % rel. Feuchte innerhalb von 24 Monaten und bei 25 °C/60 % rel. Feuchte innerhalb von 36 Monaten nicht über den kritischen Wert von 1 % steigt.

### Beispiel 5

Für die Herstellung von 1 kg einer Arzneistofflösung werden 607 g mittelkettige Triglyceride, 7,2 g Pfefferminzöl, 200 g Ethanol abs., 20 g mittelkettige Partialglyceride und 166 g Glyceroltrinitrat (GTN) in mittelkettigen Triglyceriden (als 5 %-ige Lösung, enthaltend 8,3 g GTN) eingewogen und innig gemischt. Die Lösung wird in Portionen zu 14,9 g in Flaschen aus Cycloolefinpolymer (Zeonor®) abgefüllt und die Flaschen werden mit einer Spraypumpe verschlossen. Die Zusammensetzung war bei Lagerung der Flaschen über 6 Monate bei 40 °C und 75 % relativer Luftfeuchtigkeit stabil.

## Patentansprüche

1. Pharmazeutische Zubereitung, umfassend ein Gemisch aus mindestens einem pharmazeutischen Wirkstoff, mindestens einer Alkoholkomponente und mindestens einer Triglyceridkomponente, in einem Behälter aus Kunststoff,
**dadurch gekennzeichnet, dass** der Kunststoff eine Wasserdampfdurchlässigkeit von nicht mehr als 3,0 g/m²/24h (gemessen an einer Folie von 100 µm Stärke bei 40 °C und 90 % relativer Luftfeuchte) aufweist.

2. Zubereitung nach Anspruch 1,
**dadurch gekennzeichnet, dass** der Kunststoff ein Cycloolefinpolymer (COP) ist.

3. Zubereitung nach Anspruch 1,
**dadurch gekennzeichnet, dass** der Kunststoff ein Cycloolefincopolymer (COC) ist.

4. Zubereitung nach einem der vorangehenden Ansprüche,
**dadurch gekennzeichnet, dass** die mindestens eine Alkoholkomponente aus der Gruppe Ethanol, 1-Propanol, 2-Propanol und Propylenglycol gewählt ist.

5. Zubereitung nach einem der vorangehenden Ansprüche,
**dadurch gekennzeichnet, dass** die mindestens eine Triglyceridkomponente aus der Gruppe der pflanzlichen und tierischen Fette und Öle gewählt ist.

6. Zubereitung nach einem der vorangehenden Ansprüche,
**dadurch gekennzeichnet, dass** es sich bei der mindestens einen Triglyceridkomponente um mittelkettige Triglyceride handelt.

7. Zubereitung nach einem der vorangehenden Ansprüche,
**dadurch gekennzeichnet, dass** sie ein Gemisch aus 10 bis 80 Gew.-% Ethanol, 10 bis 80 Gew.-% Triglyceride und 0,2 bis 5 Gew.-% Glyceroltrinitrat, jeweils bezogen auf das Gesamtgewicht des Gemisches, enthält.

## Claims

1. Pharmaceutical preparation, comprising a mixture of at least one pharmaceutical active ingredient, at least one alcohol component and at least one triglyceride component in a container made of plastic material, **characterized in that** the plastic has a permeability to water of not more than 3.0 g/m²/24h (measured using a film having a thickness of 100 µm at 40 °C and 90 % relative humidity).

2. Preparation according to claim 1, **characterized in that** the plastic material is a cycloolefin polymer (COP).

3. Preparation according to claim 1, **characterized in that** the plastic material is a cycloolefin copolymer (COC).

4. Preparation according to one of the previous claims, **characterized in that** the at least one alcohol component is selected from the group comprising ethanol, 1-propanol, 2-propanol, and propylene glycol.

5. Preparation according to one of the previous claims, **characterized in that** the at least one triglyceride component is selected from the group of plant and animal fats and oils.

6. Preparation according to anyone of the previous claims, **characterized in that** the at least one triglyceride component is a medium-chain triglyceride.

7. Preparation according to one of the previous claims, **characterized in that** it contains a mixture of 10 to 80 wt.-% ethanol, 10 to 80 wt.-% triglycerides and 0.2 to 5 wt.-% glycerol trinitrate, in each case basing on the total weight of the mixture.

## Revendications

1. Préparation pharmaceutique comprenant un mélange d'au moins un principe actif pharmaceutique, au moins un composant alcoolique, et au moins un composant triglycéridique, dans un récipient en matière plastique, **caractérisée en ce que** la matière plastique présente une perméabilité à la vapeur d'eau ne dépassant pas 3,0g / m² / 24h (mesurée sur un film de 100µm d'épaisseur à 40 °C et à 90% d'humidité relative).

2. Préparation selon la revendication 1, **caractérisée en ce que** la matière plastique est un polymère de cyclo-oléfine (COP).

3. Préparation selon la revendication 1, **caractérisée en ce que** la matière plastique est un copolymère de cyclo-oléfine (COC).

4. Préparation selon l'une des revendications précédentes, **caractérisée en ce que** le au moins composant alcoolique est choisi dans le groupe : Ethanol, 1-Propanol, 2-Propanol et Propylène-glycol.

5. Préparation selon l'une quelconque des revendications précédentes, **caractérisée en ce que** le au moins composant triglycéridique est choisi dans le groupe des graisses et huiles végétales et animales.

6. Préparation selon l'une quelconque des revendications précédentes, **caractérisée en ce qu'**il s'agit, pour le au moins composant triglycéridique, d'un triglycéride à chaîne moyenne.

7. Préparation selon l'une quelconque des revendications précédentes, **caractérisée en ce qu'**elle contient un mélange de 10 à 80% en poids d'Ethanol, 10 à 80% en poids de triglycéridiques et de 0,2 à 5% en poids de nitroglycérine, dans chaque cas par rapport au poids total du mélange.
